# EUROPEAN PATENT APPLICATION

(11) **EP 3 477 325 A2**
(43) Date of publication of application: **01.05.2019**
(21) Application number: 18201724.4
(22) Date of filing: 22.10.2018
(51) Int. Cl.: G01R 33/56, G06T 7/00, G16H 50/20, A61B 5/055, G01R 33/48, G01R 33/563

(54) **DECODING PATIENT CHARACTERISTICS AND BRAIN STATE FROM MAGNETIC RESONANCE IMAGING DATA**

(30) Priority: 24.10.2017 US 201715792041
(71) Applicant: Siemens Healthcare GmbH, 91052 Erlangen (DE)
(72) Inventor: Pereira, Francisco, Jersey City, NJ 07302 (US); Bin, Lou, Princeton, NJ 08540 (US); Tuysuzoglu, Ahmet, Jersey City, NJ 07306 (US); Mansi, Tommaso, Plainsboro, NJ 08536 (US); Comaniciu, Dorin, Princeton Junction, NJ 08550 (US)
(74) Representative: Patentanwälte Bals & Vogel

(57) **Abstract**

A computer-implemented method for decoding patient characteristics and brain state from multi-modality brain imaging data includes receiving a plurality of brain imaging datasets comprising brain imaging data corresponding to plurality of subjects. The brain imaging datasets are aligned to a common reference space and quantitative measures are extracted from each brain imaging dataset. Non-imaging characteristics corresponding to each subject are received and a forward model is trained to map the plurality of characteristics to the quantitative measures.

## Description

### GOVERNMENT INTERESTS

This invention was made with government support under grant FA8650-14-C-7358 awarded by Air Force Research Laboratory. The government has certain rights in the invention.

This research is based upon work supported in part by the Office of the Director of National Intelligence (ODNI), Intelligence Advanced Research Projects Activity (IARPA), via Air Force Research Laboratory (AFRL). The views and conclusions contained herein are those of the authors and should not be interpreted as necessarily representing the official policies or endorsements, either expressed or implied, of ODNI, IARPA, AFRL, or the U.S. Government. The U.S. Government is authorized to reproduce and distribute reprints for Governmental purposes notwithstanding any copyright annotation thereon.

### TECHNICAL FIELD

The present invention relates generally to methods, systems, and apparatuses for decoding patient characteristics and brain state from magnetic resonance imaging data.

### BACKGROUND

There are typically three distinct steps involved with using imaging data to answer clinical questions. First, conventional healthcare imaging systems are typically used primarily for image acquisition. Second, radiologists then examine these images and convey results to the physician referring the scan. Finally, the physician uses the results to answer the clinical question that motivated it.

One major deficiency of the three-step process described above is that the output of the scanner is primarily used for visual inspection by a radiologist. This means that any image characteristic that is not visually salient is not taken into consideration for reporting, even though such information is present in imaging data. Examples of these in the context of Magnetic Resonance Imaging (MRI) include (sub)cortical structure volumetry (derived from structural MRI), structural connectivity (derived from diffusion MRI), functional connectivity (derived from resting-state functional MRI data), and task-related activation (deriving from functional MRI), among others. All of these have shown to have diagnostic value for computational psychiatry or neurology applications, in isolation or combined with non-imaging information such as assays.

A second deficiency of three-step process of answering clinical questions is that derivation of quantitative measures from basic MRI modalities is artisanal. The generation of these quantitative measures happens as a post-processing step applied to clinical or research data, and only then can these be used in high-level image analysis for assistance in diagnosis and prognosis, often together with additional non-imaging data. Although most of this quantification is now a standard procedure routinely used by the research community, it is up to data owners to carry it out, fit models and make sense of results; this process often relies on inexperienced research trainees.

Additionally, with the three-step process of answering clinical questions, image interpretation lacks context from the patient population. What is normal or abnormal within patients with the condition of interest is determined solely by the radiologist (who may have seen few cases of that particular condition) or by the clinician (who may not look at many images). In addition to first deficiency described above, this relies on the experience of a single person, possibly augmented by consults, to both determine whether something is abnormal and to factor out patient-specific effects. For example, hippocampus size being small might be meaningless in an older patient, but be a symptom of disease in a younger one. It is also likely that patient characteristics will affect many of the quantitative measures that cannot be visualized. The only way of determining this is to tabulate their values across patient and control populations, which is impractical without software assistance (given that there might be tens to hundreds of thousands of values).

### SUMMARY

Embodiments of the present invention address and overcome one or more of the above shortcomings and drawbacks by providing methods, systems, and apparatuses related to decoding patient characteristics and brain state from magnetic resonance imaging data. The system described herein may be used, for example, with patients suspected of having or diagnosed with a neurological or psychiatric disease or condition (e.g., neurological: Alzheimer's, Parkinson's, Traumatic Brain Injury; psychiatric: Schizophrenia, Depression, Attention Deficit and Hyperactivity Disorder, Post-traumatic Stress Disorder). The system can be trained on existing imaging datasets for each disease, containing both patients and controls. In addition, it can be trained on datasets of normal subjects and their characteristics (e.g., UK Biobank or Human Connectome Project).

According to some embodiments, a computer-implemented method for decoding patient characteristics and brain state from multi-modality brain imaging data includes receiving brain imaging datasets comprising brain imaging data corresponding to plurality of subjects. These brain imaging datasets may include, for example, one or more of a 3D structural MRI dataset, a diffusion MRI dataset, a resting-state functional MRI dataset, and a task-based functional MRI dataset. The brain imaging datasets are aligned to a common reference space and quantitative measures are extracted from each brain imaging dataset. These quantitative measures may include, for example, one or more of brain structure volumes, structural connectivity between atlas brain regions, functional connectivity between atlas brain regions, activation maps for different stimuli, and activation maps for stimulus contrasts. Following alignment of the brain imaging datasets, a forward model is trained to map non-imaging characteristics corresponding to each subject to the quantitative measures.

Once the forward model is generated, it may be applied to a new brain imaging dataset corresponding to a new subject by extracting new quantitative measures from the new brain imaging dataset and then using the forward model to predict unknown characteristics corresponding to the new subject based on the new quantitative measures and known non-imaging characteristics. In one embodiment, a regression routine is applied to regress out effects of the known non-imaging characteristics from the prediction of the unknown characteristics.

The non-imaging characteristics corresponding to each subject used in the aforementioned method may include, for example, one or more of demographics information, medical history information, assay results, diagnosis information, and prognosis information. These characteristics may be received via an electronic medical record corresponding to each subject. The electronic medical record can be parsed (e.g., using techniques generally known in the art) to extract the non-imaging characteristics corresponding to each subject.

The forward model used in the aforementioned method may be trained by first for all subjects, transforming the quantitative measures from each brain imaging dataset into a quantitative measures vector. The quantitative measures vector corresponding to all the subjects is then aggregated into a quantitative measures matrix. Next, for all subjects, the non-imaging characteristics are transformed into a characteristics vector. The characteristics vector corresponding to all the subjects are aggregated into a characteristics matrix. Then, one or more regression models are learned that predict the quantitative measures matrix from the characteristics matrix. These regression models may include, for example, ridge regression models that predict each column of the quantitative measures matrix separately. In some embodiments, the ridge regression models predict each column of the quantitative measures matrix separately using generalized cross-validation to set a regularization parameter.

According to another aspect of the present invention, computer-implemented method for decoding patient characteristics and brain state from multi-modality brain imaging data includes receiving a brain imaging dataset corresponding to a subject and extracting quantitative measures from the brain imaging dataset. Known non-imaging characteristics corresponding to the subject are received. A forward model is used to predict one or more unknown characteristics corresponding to the subject based on the quantitative measures and the one or more known non-imaging characteristics.

In other embodiments, a system for decoding patient characteristics and brain state from multi-modality brain imaging data includes a magnetic resonance imaging scanner and one or more processors. The magnetic resonance imaging scanner is configured to acquire brain imaging datasets corresponding to a subject. The processors are configured to extract quantitative measures from the brain imaging dataset, and use machine learning models to predict one or more unknown characteristics corresponding to the subject based on the quantitative measures and known non-imaging characteristics corresponding to the subject.

Additional features and advantages of the invention will be made apparent from the following detailed description of illustrative embodiments that proceeds with reference to the accompanying drawings.

### BRIEF DESCRIPTION OF THE DRAWINGS

The foregoing and other aspects of the present invention are best understood from the following detailed description when read in connection with the accompanying drawing. For the purpose of illustrating the invention, there are shown in the drawing exemplary embodiments that are presently preferred, it being understood, however, that the invention is not limited to the specific instrumentalities disclosed. Included in the drawings are the following Figures:
FIG. 1 illustrates a pre-processing pipeline that may be applied in some embodiments of the present invention;
FIG. 2 illustrates a forward model that may be applied in some embodiments. As is generally understood in the art, a forward model is a model that describes the current state of the system;
FIG. 3 illustrates a method for decoding patient characteristics and brain state from multi-modality brain imaging data, according to some embodiments;
FIG. 4 illustrates a method for performing testing on new datasets, according to some embodiments; and
FIG. 5 provides an example of a parallel processing memory architecture that may be utilized to implement the machine models and other aspects of the various methods discussed herein.

### DETAILED DESCRIPTION

The following disclosure describes the present invention according to several embodiments directed at methods, systems, and apparatuses related to decoding of patient characteristics, diagnosis, and state from multi-modality brain imaging data, coupled with other information. The system can be used to answer specific clinical questions, such as: how likely it is that a patient has a certain neurological or psychiatric disease; why a patient brain may be absent visible symptoms; how the disease evolves in this patient; if they will need treatment; how a treatment is working; how a treatment is changing the brain of a particular patient; and how similar patients react to different treatments. The technology described herein answers such questions in a manner customized to the patient, taking into account their specific characteristics (e.g., age, gender, medical history, etc.).

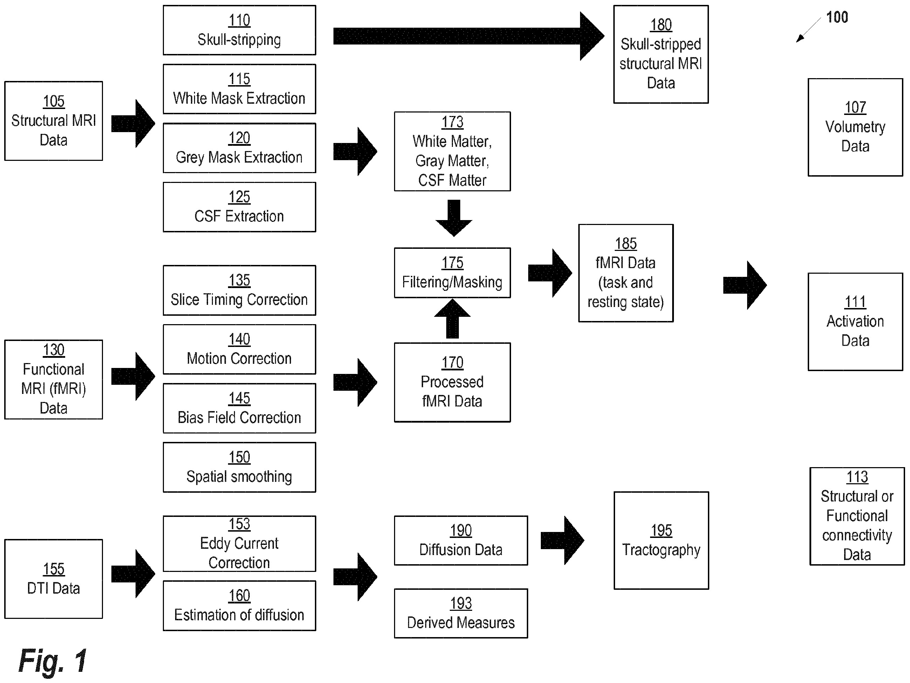

FIG. 1 illustrates a pre-processing pipeline 100 that may be applied in some embodiments of the present invention. Briefly, this pipeline 100 takes as input Structural MRI Data 105, Functional MRI Data 130 and Diffusion Tensor Imaging (DTI) Data 155. The pipeline 100 derives a series of quantitative measures for each brain imaging dataset. In this example, there are three such measures: Volumetry Data 107, Activation Data 111, and Structural/Functional Connectivity Data 113. In order to derive these quantitative measures, a series of processes generally known in the art are combined as described in further detail below.

Structural MRI Data 105 is processed by a Skull-stripping Process 110 to generate Skull-Stripping MRI Data 180 The Structural MRI Data 105 is also processed by a White Matter Mask Extraction Process 115, Gray Matter Mask Extraction Process 120, and Cerebrospinal Fluid (CSF) Mask Extraction Process 125 to yield a Brain Matter Dataset 173 of white matter, gray matter, and CSF included in the Structural MRI Data 105.

Functional MRI Data 130 from a plurality of fMRI acquisitions are processed using a Slice Timing Correction Data Process 135, a Motion Correction Data Process 140, a Bias Field Correction Data Process 145, and a Spatial Smoothing Data Process 150 to yield Processed fMRI Data 170. A Filtering/Masking Process 175 is applied to the Brain Matter Dataset 115 and the Processed fMRI Data 170 to generate fMRI Data 185 corresponding to tasks performed by the subjects and the subjects' resting state.

An Eddy Current Correction Process 153 and an Estimation of a Diffusion Model Process 160 are applied to DTI Data 155 which is used to generate Diffusion Data 190 and Derived Measures 193 such as track weighted images (TWI) and fractional anisotropy (FA). Additionally, a Tractography Process 195 is performed on the Diffusion Data 190.

Finally, using all of the derived information generated by the processes in FIG. 1, the Volumetry Data 107, Activation Data 111, and Structural/Functional Connectivity Data 113 are generated. Volumetry Data 107 is generated from structural images, by first segmenting them into various cortical and subcortical structures, and then estimating the volume of those structures. Another possible volumetric measurement is cortical thickness, obtained by first segmenting the cortical surface and then estimating its thickness. Activation Data 111 is derived from functional MRI data using general linear model (GLM) that capture contrasts of interest (e.g., between baseline and task performance, or different task conditions). The Structural/Functional Connectivity Data 113 is generated by first specifying points or regions of interest, and then calculating the degree to which they are connected. For functional connectivity, this will usually be a correlational measure comparing time courses of voxels or regions during functional scanning of the subject at rest. For structural connectivity, this will usually be an estimation of the number of tracts between voxels or regions, derived from the tractography process. All of these techniques are generally known in the art, and can be implemented, for example, with open source software.

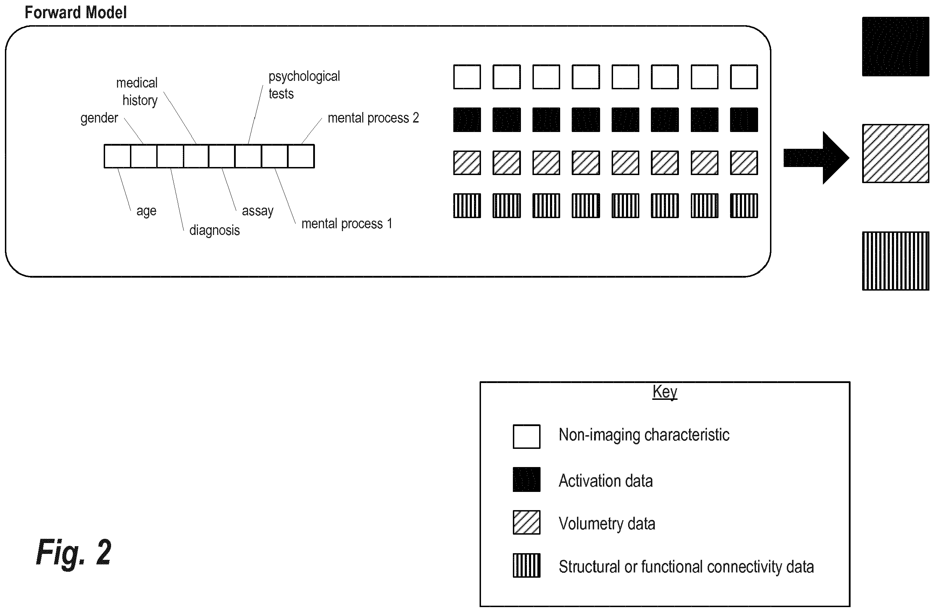

FIG. 2 illustrates a forward model that may be applied in some embodiments. As is generally understood in the art, a forward model is a model that describes the current state of the system. Known mathematics associated with the system are used to decide how the system will behave under different scenarios. The techniques described herein learn a forward model that maps all of the characteristics available for a subject to all the quantitative measures derived from its imaging data. In the example of FIG. 2, the characteristics include age, gender, diagnosis, medical history, assay results, psychological tests, and two mental processes. The forward model operates on new test subjects by first taking as input the quantitative data from the new test subjects and then generating a prediction of any characteristic of interest, factoring out the influence of other characteristics. For example, it will predict whether a patient has the disease -- diagnostic characteristic -- given the imaging data, taking into account the age, gender, and whatever other characteristic is known.

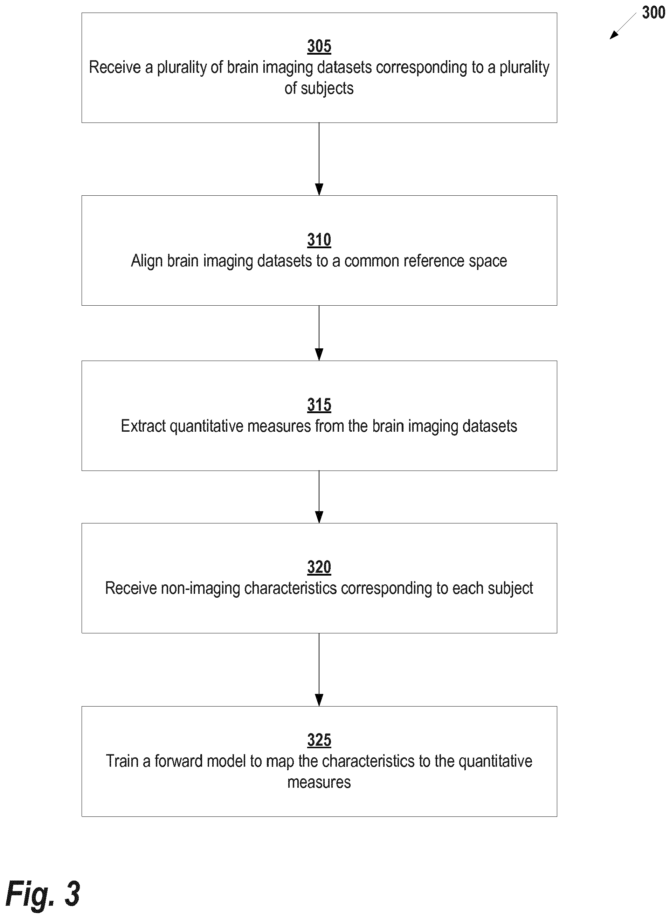

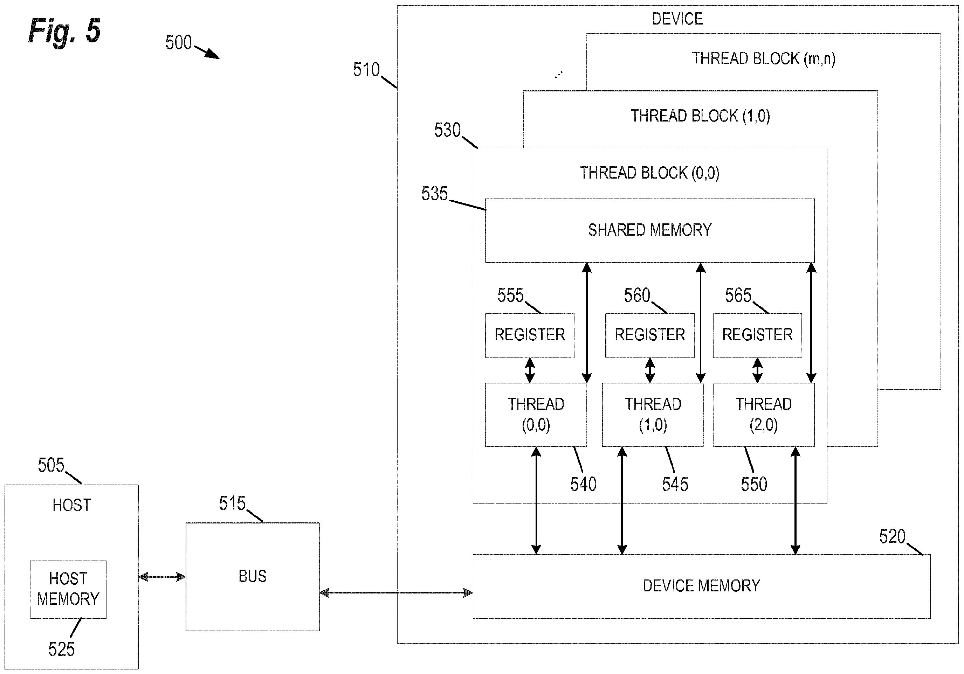

FIG. 3 illustrates a method 300 for decoding patient characteristics and brain state from multi-modality brain imaging data, according to some embodiments. This method 300 is intended to be performed using a computing system, either local to the scanner acquiring the imaging data or on a computing system remote from the scanner. In some embodiments, the computing system described below with respect to FIG. 5 is employed.

Starting at step 305, a plurality of brain imaging datasets are received by the computing system. These datasets comprise brain imaging data corresponding to plurality of subjects. The datasets may include, for example, one or more of a 3D structural MRI dataset, a diffusion MRI dataset, a resting-state functional MRI dataset, and a task-based functional MRI dataset. Next at step 310, the brain imaging datasets are aligned to a common reference space, and thence to a standardized space (e.g., Molecular NeuroImaging) that can be compared across subjects and studies. In the process, the brain may be segmented according to different atlases (e.g., Gordon 2014). The alignment to a common reference space, for a given subject, is carried out by computing a registration operation between the individual 3D structural MRI and a template 3D structural MRI (publicly available, derived from hundreds of patients, accompanied by brain atlases annotating which structure each voxel belongs to). This operation comprises a linear registration step and a nonlinear warp step. This alignment procedure is generally known in the art and can be implemented, for example, with open source software.

A plurality of quantitative measures is extracted from each brain imaging dataset at step 315. These quantitative measures may include, for example, one or more of brain structure volumes, structural connectivity between atlas brain regions, functional connectivity between atlas brain regions, activation maps for different stimuli, and activation maps for stimulus contrasts. Next, at step 320, the computing system receives a plurality of non-imaging characteristics corresponding to each subject. The non-imaging characteristics may include, for example, one or more of demographics information, medical history information, assay results, diagnosis information (e.g., does the subject have the disease? disease load?), and prognosis information (e.g., time to onset of symptoms). In some embodiments, the computing system receives the non-imaging characteristics in one or more electronic medical records (EMR) and extracts the relevant characteristics using one or more parsing techniques generally known in the art.

Continuing with reference to FIG. 3, at step 325, the computing system trains a forward model to map the characteristics to the quantitative measures. Various techniques may be used for training the forward model. For example, in some embodiments, training of the forward model is performed as follows. First, for all subjects, the quantitative measures are transformed into a vector thereby yielding a matrix X defined as a number of subjects by the number of measures. Secondly, for all subjects, the subject characteristics are transformed into a vector to yield a matrix Z defined as a number of subjects by the number of characteristics. Third, a regression model B is learned that predicts X from Z. In one embodiment, ridge regression models are learned that predict each column of X separately, using generalized cross-validation to set the regularization parameter. For ease of description it may be assumed that all quantitative measures and characteristics are present for all subjects. If not, matrix completion may be used to predict missing entries; this can be implemented, for example, using one or more dictionary learning techniques general known in the art.

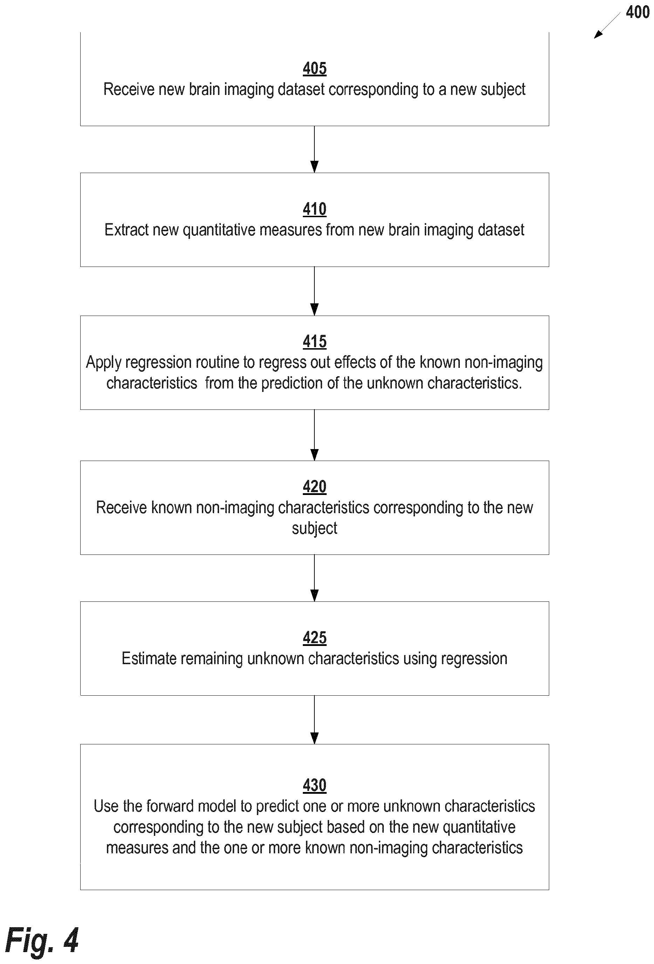

FIG. 4 illustrates a method 400 for performing testing on new datasets, according to some embodiments. The inputs used during testing are whatever imaging quantitative measures are available. For the purposes of this discussion, assume that the measures are stored in a matrix *Xtest* sized as the number of subjects by the number of the measures. The inputs further include known subject characteristics (e.g., age, gender, medical history). These characteristics may be stored in a matrix *Ctest* sized as the number of subjects by the number of known characteristics.

Starting at step 405 the computing system receives a new brain imaging datasets corresponding to a new subject and, at step 410, the computing system extracts new quantitative measures from the received dataset. At step 415, a regression routine is applied to regress out effects of the known non-imaging characteristics (e.g., age, gender) from the prediction of the unknown characteristics. More formally, we remove the effect from the quantitative measures *Xtest* by *Xtest* = *Xtest* - *Ctest* * B(*crange*,:), where crange are the rows of *B* containing the regression weights from those known characteristics. Next, at step 420, the computing system receives one or more known non-imaging characteristics corresponding to the new subject. At step 425, the remaining unknown characteristics Z are estimated by regressing X from B(*zrange*,:), where *zrange* are the rows of *B* containing the regression weights from the unknown characteristics. More formally, this is *Xtest'* ∼ B(*zrange*)' *Zestimate'.* In some embodiments, the regression applied at steps 415 and 425 may each be carried out using ridge regression models. Then, at step 430, the computing system uses the forward model to predict one or more unknown characteristics corresponding to the new subject based on the new quantitative measures and the one or more known non-imaging characteristics. That is, the desired predictions are extracted from *Zestimate.*

As described above, the methods 300 and 400 described in FIGS. 3 and 4 can be applied to quantitative measures for new patients and predict a characteristic (e.g., diagnosis, time to onset of symptoms, disease load, etc.). It will produce an automated report that may be presented in a graphical user interface (GUI) to detail the rationale for the decision (e.g., which imaging measures determined the prediction), in an interpretable form such as a decision rule with a confidence estimate. Additionally, the report can place the patient in context of comparable patients or controls, with most probable explanations for findings

In some embodiments, the methods 300 and 400 described in FIGS. 3 and 4 are integrated (either locally or remotely) in an image scanner. Given a particular clinical question of interest, the scanner may perform various operations based on the model and available data. For example, the scanner may determine the appropriate modalities to scan and guide an operator in collecting data. Next, the scanner may feed the resulting data to the system, together with relevant patient information. Then, the scanner may obtain the system automated report and answer to the clinical question. Optionally, in some embodiments, the scanner autonomously collects additional data to refine the answer or rule out possible explanations for findings. Thus, the intelligent scanner augments the capabilities of the clinician, by allowing them to make decisions based on information from a vast number of patients (larger than any individual center might have, or that an individual expert could see during their career). It would also facilitate personalized treatment based on imaging data in conjunction with patient data.

The technology described herein envisages a future where brain scanning is a necessary part of treatment, monitoring, and prevention of neurological and psychiatric diseases. At a broader level, the system is meant to quantify the meaningful use of a test ordered by the physician. Hence, it supports clinical workflows and decision making, and brings into the scanner parts of those workflows that are now provided by third parties, or inaccessible to providers not engaged in clinical research. Therefore, it augments the capabilities of the clinician, by allowing them to make decisions based on information from a vast number of patients (larger than any individual center might have, or than the experience of any individual). It will also facilitate personalized treatment based on imaging data in conjunction with patient data. From that perspective, it should also be of interest to various healthcare entities in general, given that it could be provided as part of broader healthcare provision solutions.

FIG. 5 provides an example of a parallel processing platform 500 that may be utilized to implement the machine learning models and other aspects of the various processes discussed herein. This platform 500 may be used in embodiments of the present invention where NVIDIA CUDA™ (or a similar parallel computing platform) is used. The architecture includes a host computing unit ("host") 505 and a graphics processing unit (GPU) device ("device") 510 connected via a bus 515 (e.g., a PCIe bus). The host 505 includes the central processing unit, or "CPU" (not shown in FIG. 5), and host memory 525 accessible to the CPU. The device 510 includes the graphics processing unit (GPU) and its associated memory 520, referred to herein as device memory. The device memory 520 may include various types of memory, each optimized for different memory usages. For example, in some embodiments, the device memory includes global memory, constant memory, and texture memory.

Parallel portions of a big data platform and/or big simulation platform may be executed on the platform 500 as "device kernels" or simply "kernels." A kernel comprises parameterized code configured to perform a particular function. The parallel computing platform is configured to execute these kernels in an optimal manner across the platform 500 based on parameters, settings, and other selections provided by the user. Additionally, in some embodiments, the parallel computing platform may include additional functionality to allow for automatic processing of kernels in an optimal manner with minimal input provided by the user.

The processing required for each kernel is performed by a grid of thread blocks (described in greater detail below). Using concurrent kernel execution, streams, and synchronization with lightweight events, the platform 500 of FIG. 5 (or similar architectures) may be used to parallelize portions of the model based operations performed in training or utilizing the smart editing processes discussed herein. For example, in embodiments where a convolutional neural network is used as the machine learning model, the platform 500 can be used to perform operations such as forward and backward convolution, pooling, normalization, etc. Additionally, the parallel processing platform 500 may be used to execute multiple instances of a machine learning model in parallel. For example, multiple instances of the forward model described above with respect to FIG. 3 may be executed in parallel with different parameters to simultaneously train on different datasets.

The device 510 includes one or more thread blocks 530 which represent the computation unit of the device 510. The term thread block refers to a group of threads that can cooperate via shared memory and synchronize their execution to coordinate memory accesses. For example, in FIG. 5, threads 540, 545 and 550 operate in thread block 530 and access shared memory 535. Depending on the parallel computing platform used, thread blocks may be organized in a grid structure. A computation or series of computations may then be mapped onto this grid. For example, in embodiments utilizing CUDA, computations may be mapped on one-, two-, or three-dimensional grids. Each grid contains multiple thread blocks, and each thread block contains multiple threads. For example, in FIG. 5, the thread blocks 530 are organized in a two dimensional grid structure with *m*+1 rows and *n*+1 columns. Generally, threads in different thread blocks of the same grid cannot communicate or synchronize with each other. However, thread blocks in the same grid can run on the same multiprocessor within the GPU at the same time. The number of threads in each thread block may be limited by hardware or software constraints.

Continuing with reference to FIG. 5, registers 555, 560, and 565 represent the fast memory available to thread block 530. Each register is only accessible by a single thread. Thus, for example, register 555 may only be accessed by thread 540. Conversely, shared memory is allocated per thread block, so all threads in the block have access to the same shared memory. Thus, shared memory 535 is designed to be accessed, in parallel, by each thread 540, 545, and 550 in thread block 530. Threads can access data in shared memory 535 loaded from device memory 520 by other threads within the same thread block (e.g., thread block 530). The device memory 520 is accessed by all blocks of the grid and may be implemented using, for example, Dynamic Random-Access Memory (DRAM).

Each thread can have one or more levels of memory access. For example, in the platform 500 of FIG. 5, each thread may have three levels of memory access. First, each thread 540, 545, 550, can read and write to its corresponding registers 555, 560, and 565. Registers provide the fastest memory access to threads because there are no synchronization issues and the register is generally located close to a multiprocessor executing the thread. Second, each thread 540, 545, 550 in thread block 530, may read and write data to the shared memory 535 corresponding to that thread block 530. Generally, the time required for a thread to access shared memory exceeds that of register access due to the need to synchronize access among all the threads in the thread block. However, like the registers in the thread block, the shared memory is typically located close to the multiprocessor executing the threads. The third level of memory access allows all threads on the device 510 to read and/or write to the device memory 520. Device memory 520 requires the longest time to access because access must be synchronized across the thread blocks operating on the device. Thus, in some embodiments, each brain imaging dataset can be divided into segments using data locality techniques generally known in the art. Then, each segment can be processed in parallel using register memory, with shared and device memory only being used as necessary to combine the results to provide the results for the complete dataset.

The embodiments of the present disclosure may be implemented with any combination of hardware and software. For example, aside from parallel processing architecture presented in FIG. 5, standard computing platforms (e.g., servers, desktop computer, etc.) may be specially configured to perform the techniques discussed herein. In addition, the embodiments of the present disclosure may be included in an article of manufacture (e.g., one or more computer program products) having, for example, computer-readable, non-transitory media. The media may have embodied therein computer readable program code for providing and facilitating the mechanisms of the embodiments of the present disclosure. The article of manufacture can be included as part of a computer system or sold separately.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

An executable application, as used herein, comprises code or machine readable instructions for conditioning the processor to implement predetermined functions, such as those of an operating system, a context data acquisition system or other information processing system, for example, in response to user command or input. An executable procedure is a segment of code or machine readable instruction, sub-routine, or other distinct section of code or portion of an executable application for performing one or more particular processes. These processes may include receiving input data and/or parameters, performing operations on received input data and/or performing functions in response to received input parameters, and providing resulting output data and/or parameters.

A graphical user interface (GUI), as used herein, comprises one or more display images, generated by a display processor and enabling user interaction with a processor or other device and associated data acquisition and processing functions. The GUI also includes an executable procedure or executable application. The executable procedure or executable application conditions the display processor to generate signals representing the GUI display images. These signals are supplied to a display device which displays the image for viewing by the user. The processor, under control of an executable procedure or executable application, manipulates the GUI display images in response to signals received from the input devices. In this way, the user may interact with the display image using the input devices, enabling user interaction with the processor or other device.

The functions and process steps herein may be performed automatically or wholly or partially in response to user command. An activity (including a step) performed automatically is performed in response to one or more executable instructions or device operation without user direct initiation of the activity.

The system and processes of the figures are not exclusive. Other systems, processes and menus may be derived in accordance with the principles of the invention to accomplish the same objectives. Although this invention has been described with reference to particular embodiments, it is to be understood that the embodiments and variations shown and described herein are for illustration purposes only. Modifications to the current design may be implemented by those skilled in the art, without departing from the scope of the invention. As described herein, the various systems, subsystems, agents, managers and processes can be implemented using hardware components, software components, and/or combinations thereof. No claim element herein is to be construed under the provisions of 35 U.S.C. 112(f) unless the element is expressly recited using the phrase "means for."

## Claims

1. A computer-implemented method for decoding patient characteristics and brain state from multi-modality brain imaging data, the method comprising:
receiving a plurality of brain imaging datasets comprising brain imaging data corresponding to plurality of subjects;
aligning the plurality of brain imaging datasets to a common reference space;
extracting a plurality of quantitative measures from each brain imaging dataset;
receiving a plurality of non-imaging characteristics corresponding to each subject; and
training a forward model to map the plurality of non-imaging characteristics to the plurality of quantitative measures.

2. The method according to claim 1, further comprising:
receiving a new brain imaging dataset corresponding to a new subject;
extracting a plurality of new quantitative measures from the new brain imaging dataset;
receiving one or more known non-imaging characteristics corresponding to the new subject; and
using the forward model to predict one or more unknown characteristics corresponding to the new subject based on the plurality of new quantitative measures and the one or more known non-imaging characteristics.

3. The method according to claim 2, wherein a regression routine is applied to regress out effects of the known non-imaging characteristics from the prediction of the one or more unknown characteristics.

4. The method according to any of the preceding claims, wherein the plurality of brain imaging datasets comprise one or more of a 3D structural MRI dataset, a diffusion MRI dataset, a resting-state functional MRI dataset, and a task-based functional MRI dataset.

5. The method according to any of the preceding claims, wherein the plurality of quantitative measures comprise one or more of brain structure volumes, structural connectivity between atlas brain regions, functional connectivity between atlas brain regions, activation maps for different stimuli, and activation maps for stimulus contrasts.

6. The method according to any of the preceding claims, wherein the plurality of non-imaging characteristics corresponding to each subject comprise one or more of demographics information, medical history information, assay results, diagnosis information, and prognosis information.

7. The method according to any of the preceding claims, wherein receiving the plurality of non-imaging characteristics corresponding to each subject comprises:
receiving an electronic medical record corresponding to each subject; and
parsing each electronic medical record to extract the non-imaging characteristics corresponding to each subject.

8. The method according to any of the preceding claims, wherein the forward model is trained by a process comprising:
for all subjects, transform the plurality of quantitative measures from each brain imaging dataset in to a quantitative measures vector;
aggregating the quantitative measures vector corresponding to all the subjects into a quantitative measures matrix;
for all subjects, transform the plurality of non-imaging characteristics into a characteristics vector;
aggregating the characteristics vector corresponding to all the subjects into a characteristics matrix; and
learning one or more regression models that predict the quantitative measures matrix from the characteristics matrix.

9. The method according to claim 8, wherein the one or more regression models comprise ridge regression models that predict each column of the quantitative measures matrix separately.

10. The method according to claim 9, wherein the ridge regression models predict each column of the quantitative measures matrix separately using generalized cross-validation to set a regularization parameter.

11. A computer-implemented method for decoding patient characteristics and brain state from multi-modality brain imaging data, the method comprising:
receiving a brain imaging dataset corresponding to a subject;
extracting a plurality of quantitative measures from the brain imaging dataset;
receiving one or more known non-imaging characteristics corresponding to the subject; and
using a forward model to predict one or more unknown characteristics corresponding to the subject based on the plurality of quantitative measures and the one or more known non-imaging characteristics.

12. The method according to claim 11, wherein a regression routine is applied to regress out effects of the known non-imaging characteristics from the prediction of the one or more unknown characteristics.

13. The method according to claims 11 or 12, further comprising:
receiving a plurality of brain imaging datasets comprising brain imaging data corresponding to plurality of subjects;
aligning the plurality of brain imaging datasets to a common reference space;
extracting a plurality of quantitative measures from each brain imaging dataset;
receiving a plurality of non-imaging characteristics corresponding to each subject;
training the forward model to map the plurality of characteristics to the plurality of quantitative measures.

14. The method according to claim 13, wherein the forward model is trained by a process comprising:
for all subjects, transform the plurality of quantitative measures from each brain imaging dataset in to a quantitative measures vector;
aggregating the quantitative measures vector corresponding to all the subjects into a quantitative measures matrix;
for all subjects, transform the plurality of non-imaging characteristics into a characteristics vector;
aggregating the characteristics vector corresponding to all the subjects into a characteristics matrix; and
learning one or more regression models that predict the quantitative measures matrix from the characteristics matrix.

15. The method according to claim 14, wherein the one or more regression models comprise ridge regression models that predict each column of the quantitative measures matrix separately and the ridge regression models predict each column of the quantitative measures matrix separately using generalized cross-validation to set a regularization parameter.

16. The method according to claim 13 or 14, wherein the plurality of brain imaging datasets comprise one or more of a 3D structural MRI dataset, a diffusion MRI dataset, a resting-state functional MRI dataset, and a task-based functional MRI dataset.

17. The method according to any of the preceding claims 13 to 16, wherein the plurality of quantitative measures comprise one or more of brain structure volumes, structural connectivity between atlas brain regions, functional connectivity between atlas brain regions, activation maps for different stimuli, and activation maps for stimulus contrasts.

18. The method according to any of the preceding claims 13 to 17, wherein the plurality of non-imaging characteristics corresponding to each subject comprise one or more of demographics information, medical history information, assay results, diagnosis information, and prognosis information.

19. The method according to any of the preceding claims 13 to 18, wherein receiving the plurality of non-imaging characteristics corresponding to each subject comprises:
receiving an electronic medical record corresponding to each subject; and
parsing each electronic medical record to extract the non-imaging characteristics corresponding to each subject.

20. A system for decoding patient characteristics and brain state from multi-modality brain imaging data, the system comprising:
a magnetic resonance imaging scanner configured to acquire a brain imaging datasets corresponding to a subject;
one or more processors configured to:
extract a plurality of quantitative measures from the brain imaging dataset, and
use one or more machine learning models to predict one or more unknown characteristics corresponding to the subject based on the plurality of quantitative measures and one or more known non-imaging characteristics corresponding to the subject.
